# EUROPEAN PATENT APPLICATION

(11) **EP 2 676 667 A1**
(43) Date of publication of application: **25.12.2013**
(21) Application number: 13185307.9
(22) Date of filing: 08.05.2006
(51) Int. Cl.: A61K 31/765, A61P 1/10, A61K 33/00, A61K 9/20

(54) **Polyethylene glycol colonic purgative composition**

(30) Priority: 06.05.2005 US 678181 P
(62) Divisional of application: 06770123.5
(71) Applicant: Salix Pharmaceuticals, Inc., Morrisville, NC 27560 (US)
(72) Inventor: Skiendzielewski, Stephen, Norristown, PA 19403 (US); Apple, Robert, Newtown, PA 18940 (US); Rose, Martin, Bethesda, MD 20817 (US)
(74) Representative: Adam, Holger

(57) **Abstract**

This invention relates to novel colonic purgative compositions of polyethylene glycol having an average molecular weight of at least 1,000 or at least 4,000. Further, this invention relates to methods of using the colonic purgative compositions. The formulations and methods of this invention are particularly useful to cleanse the bowel prior to diagnostic and surgical procedures and can also be employed in lower dosages as a laxative to promote elimination and/or to relieve constipation.

## Description

### RELATED APPLICATIONS

This application claims priority from US Provisional Application Serial Number: 60/678,181, filed May 6, 2005, and is hereby incorporated by reference in its entirety.

### FIELD

This invention relates to colonic purgative formulations comprising at least one polyethylene glycol and uses of these formulations. In certain embodiments of this invention, the formulation consists essentially of polyethylene glycol 8000, which aids in patient tolerance and endoscope insertion. The formulation may be administered in a variety of dosage forms including, but not limited to, a solid or liquid dosage form. In certain embodiments, the composition is useful in purging the colon completely. In other embodiments, the composition is useful as a laxative.

### BACKGROUND

It is desirable to identify compounds that sufficiently cleanse the colon, but are also tolerable to the patient and do not cause adverse side effects. It is also desirable to identify compounds that are used to treat constipation and promote fecal elimination, for instance, but that can be used for sustained periods of time and do not produce uncomfortable or embarrassing side effects, such as gas. Completely clearing the bowel of fecal debris is a prerequisite before a variety of diagnostic and surgical procedures. Cleansing is important, for instance, in order to sufficiently view the gross or microscopic appearance of the colon during colonoscopy. However, the cleansing procedure must also be tolerable to patients so that they are fully compliant with the cleansing process. Poor bowel preparation, due to lack of patient compliance or insufficient cleansing, impacts the efficiency and cost of these procedures, especially if they must be repeated (Rex et al. (2002) Am. J. Gastroenterol. 97:1696-1700). Further, patients may not elect to undergo uncomfortable diagnostic procedures, which would significantly reduce early detection of disorders and increase medical costs (Harewood et al. (2002) Am. J. Gastroenterol. 97:3186-3194).

Constipation is a common disorder, with a prevalence of approximately 1.2%, and is responsible for approximately 2.5 million physician visits in the United States per year (Andorsky et al. (1990) Am. J. Gastroenterol. 85:261-265). While various treatments have been developed, many patients are unable to obtain satisfactory results with minimal or no side effects. For instance, increasing the intake of fiber or bulk laxatives fails to normalize bowel habit in up to 40% of patients, and their use may cause certain side effects such as abdominal pain, bloating, or gas (Attar et al. (1999) Gut 44:236-230). Commonly used osmotic agents, including sugars, sugar-alcohols, and polysaccharides, can be fermented by the intestinal flora (Attar et al. (1999) Gut 44:236-230). The formation of explosive gases during the fermentation process is an undesirable property during certain surgical and diagnostic procedures involving the colon, such as during a colonoscopy using equipment that may produce a spark. In some documented cases, the presence of these gases during colon electrosurgery *has* led *to* explosion *(*DeWitt et al. (1996) J. R. Coll. Surg. Edinb. 41:419). Gases produced during the use of a laxative can also be unpleasant and embarrassing.

Colonic cleansing is commonly accomplished using lavage with polyethylene glycol 3350-electrolyte solutions. A down-side of this method is that patients are required to ingest a significant amount of liquid volume within a short period of time for purgation. For instance, patients may have to ingest four liters of solution within a period of two to three hours (Afridi et al. (1995) Gastrointest. Endosc. 41:485-489). A large number of patients experience significant volume-related discomfort and adverse side effects such as nausea, cramping, and vomiting (Dipalma et al. (2003) Am. J. Gastroenterol. 98:2187-2191). Another drawback of these preparations is their salty taste, which may also lead to patient noncompliance and adverse effects. Attempts have been made to make the taste more palatable, for instance by flavoring or reducing salt content. However, these changes did not make the regimen more acceptable to the patient, nor was there an improvement in the quality of colon cleansing (Church (1998) Dis. Colon Rectum 41:1223-1225). Such preparations deter patients from colon cancer screening (Harewood et al. (2002) Am. J. Gastroenterol. 97:3186-3194).

Additionally, there are further disadvantages. Polyethylene glycol 3350-electrolyte solutions are packaged in a single four-liter jug, as a powder for reconstitution. This four-liter jug, once filled with water, is very heavy and difficult to lift, especially for elderly, infirm, or handicapped individuals. Furthermore, the visual appearance of such a large jug is psychologically daunting as the patient often feels it is impossible to consume all of the contents of such a large jug. Thus, there is a need for a less daunting option for patients, and jugs that are easier to handle. Furthermore, there is a need for a container that is easy to store in the refrigerator, as the chilled solution is often viewed as more palatable. The currently employed containers are difficult to store.

Some products currently on the market employ a combination of polyethylene glycol 3350-electrolyte solutions with another purgative, such as a stimulant purgative. There is a need for a convenient and less daunting product that does not require the addition of another active agent. The use of two types of laxatives (i.e., osmotic laxatives, such as polyethylene glycol and electrolytes, with stimulant laxatives) can increase the risk for side effects, irritation, etc. For example, the addition of a stimulant laxative to either a combination product or combination therapy regimen may increase the chance of unpleasant side effects such as stomach ache, cramping, vomiting, etc. Therefore it is desirable to have a product that does not require administration of a stimulant laxative.

Thus, there is need for colonic purgative compositions that can be tolerated by the patient, while also providing quality preparation of the bowel. Further, it is desirable that the composition provide improved bowel motility without adverse effects on the bowel.

It is also desirable to identify a preparation that could be produced easily and administered in either a solid or liquid dosage form. In addition, it is desirable to identify a preparation that can be used either as a complete purgative or as a laxative for mild catharsis, depending on the dosage administered. Such a dual function composition would be very beneficial.

### SUMMARY

The present invention relates to colonic purgative formulations; comprising polyethylene glycol and methods of their use. In one embodiment, the colonic purgative formulation consists essentially of polyethylene glycol with an average molecular weight of at least 1000. In another embodiment, the polyethylene glycol has an average molecular weight of from 4000 to 20,000. In other embodiments, the polyethylene glycol is PEG 8000. in yet another embodiment, the polyethylene glycol is PEG 3350.

In an additional embodiment, a single dosage of the colonic purgative formulation comprising from 10 to 1000 g of PEG 8000. For example, with about 10 to about 100 g for a laxative effect and about 100 to about 1000 g for a complete purgative effect.

The formulation is a dual function composition. Thus, the present invention encompasses methods of treating gastrointestinal disorders, such as constipation, by providing the colonic purgative compositions as a laxative. The present invention also encompasses methods of complete purgation in order to prepare the colon for a colonoscopy or surgical procedure, by providing higher doses of the compositions as a complete purgative. Further, the present invention encompasses methods of maintaining the elimination or promoting the elimination of feces from the bowel by providing a colonic purgative composition.

The formulation may be administered in a variety of dosage forms. In one embodiment, the formulation is dispersed or dissolved in an aqueous substance, such as water. Such a product may be packaged as a dry powder, tablet(s), or concentrated solution for reconstitution or dilution by the patient. The product may also be packaged as a dilute solution ready for consumption by the patient without the need to add additional liquid. In another embodiment the formulation is administered in a solid dosage form. Solid dosage forms include, but are not limited to, tablets, capsules, suppositories, caplets, and sachets.

Additional objects, advantages and embodiments, are set forth infra.

It is to be understood that both the foregoing general description and the following detailed description are exemplary and explanatory only and are not restrictive.
Further embodiments of the present invention are the following:
1. A colonic purgative formulation comprising a therapeutically effective amount of polyethylene glycol having an average molecular weight of at least 4000 and optional electrolytes.
2. The formulation of clause 1, wherein the polyethylene glycol comprises an average molecular weight of from 4000 to 20000.
3. The formulation of clause 1, wherein the polyethylene glycol comprises PEG 8000.
4. A daily dosage of the formulation of clause 3 for use as a laxative, comprising from 10 to 100 g of PEG 8000 and optional electrolytes.
5. A daily dosage of the formulation of clause 3 for use as a complete purgative, comprising from 100 to 1000 g of PEG 8000 and optional electrolytes.
6. A kit comprising the colonic purgative formulation of clause 1.
7. A method of purging the colon of a patient comprising administering to the patient the colonic purgative formulation of clause 1, and allowing said formulation to purge the colon.
8. The method of clause 7, wherein the purging of the colon results in partial purgation for laxative purposes.
9. The method of clause 7, wherein the purging of the colon results in complete purgation for surgical or diagnostic purposes.
10. A method of purging the colon of a patient, comprising:
   (a) administering to the patient the colonic purgative formulation of clause 1; and
   (b) maintaining the elimination or promoting the elimination of feces in the bowel.
11. A method of treating a patient with, or susceptible to, a gastrointestinal disorder, comprising:
   (a) administering to the patient the colonic purgative formulation of clause 1; and
   (b) maintaining or promoting the elimination of feces in the bowel.
12. The method of clause 11, wherein the gastrointestinal disorder is constipation.
13. A method of treating a patient suffering from, or susceptible to, constipation due to administration of a medication which causes constipation as a side effect in some patients, comprising:
   (a) administering to the patient the colonic purgative formulation of clause 1; and
   (b) maintaining or promoting the elimination of feces in the bowel.
14. The method of clause 13, wherein the medication is chosen from antacids that contain aluminum; antidepressants; blood pressure medications; calcium channel blockers; calcium supplements; chemotherapy medications; cold medicines; antihistamines; diuretics; iron supplements; medications for Parkinson's disease; lipid-lowering agents; pain medications; opiates; codeine; and tranquilizers.
15. A method of treating a patient suffering from, or susceptible to, constipation, comprising:
   (a) administering to the patient the colonic purgative formulation of cause 1; and
   (b) maintaining or promoting the elimination of feces in the bowel, wherein the constipation is due to at least one of travel; change in daily routine; lack of exercise; immobility caused by injury, illness, or aging; dehydration; irritable bowel syndrome; pregnancy; diabetes; hypothyroidism; hypercalcemia; cancer of the colon or rectum; uterine prolapse; vaginal vault prolapse; rectal prolapse; scarring from surgery; injury of the colon or rectum; Parkinson's disease; multiple sclerosis; stroke; hemorrhoids or anal fissures; delaying bowel movements; anxiety; depression; eating disorders; and obsessive-compulsive disorder.
16. The method of any one of clauses 7, 10, 11, 13, or 15, wherein the patient is administered the colonic purgative formulation in at least one application.
17. The method of any one of clauses 7, 10, 11, 13, or 15, wherein the administration occurs orally, rectally, through a feeding tube, or through a nasogastric tube.
18. The method of any one of clauses 7, 10, 11, 13, or 15, wherein the polyethylene glycol is dispersed or dissolved in an aqueous medium.
19. The method of any one of clauses 7, 10, 11, 13, or 15, wherein the polyethylene glycol is administered in a solid form.
20. The method of any one of clauses 7, 10, 11, 13, or 15, wherein the colonic purgative formulation consists essentially of from 10 to 1000 g of polyethylene glycol per unit dose and optional electrolytes.
21. A kit comprising two, two-liter containers, wherein each two-liter container contains a colonic purgative formulation of polyethylene glycol having an average molecular weight of at least 1000.
22. The kit of clause 21, wherein the polyethylene glycol has an average molecular weight of from 3000 to 8000.
23. The kit of clause 22, wherein the polyethylene glycol has an average molecular weight of 3350.
24. The kit of clause 22, wherein the polyethylene glycol has an average molecular weight of 8000.
25. The kit of clause 21, further comprising at least one electrolyte in each two-liter container.
26. The kit of clause 21, further comprising a flavoring agent in each two-liter container.
27. The kit of clause 23, wherein the polyethylene glycol is present in a dose of from 100 to 300 g in each container.
28. The kit of clause 23, wherein the two, two-liter containers are packaged. together in a box, a bag, or shrink wrap.
29. A method of complete purgation of the colon of a patient, comprising:
   (a) reconstituting or dissolving the colonic purgative formulation of clause 21 with an aqueous substance;
   (b) orally ingesting the liquid colonic purgative formulation; and
   (c) allowing said formulation to purge the colon.
30. The method of clause 29, further comprising supplying the patient with the kit of clause 21.

### DETAILED DESCRIPTION

### A. Definitions

In order that the present invention may be more readily understood, certain terms are first defined. Additional definitions are set forth throughout the detailed description.

The term **"purgative"** refers to any substance that promotes defecation. Thus, the term purgative encompasses a range of cathartic effects. For instance, the term purgative encompasses mild catharsis, producing laxation ("partial purgation"), as well as stronger catharsis, providing complete or near-complete emptying of the large bowel ("complete purgation"). In one embodiment, the term refers to diarrhea. In another embodiment, the term refers to a softening or loosening of the feces or laxation. Unless modified by "partial" or "complete," purgative or purgation encompasses the full range of purgative processes, including both complete purgation and laxation ("partial purgation").

The term **"osmotic"** refers to any substance that promotes the passage of a solvent from a solution of lesser to one of greater solute concentration when the two solutions are separated by a membrane that selectively prevents the passage of solute molecules, but is permeable to the solvent. In the present invention, the term "osmotic" may refer to the ability of a substance to draw water into the intestines.

The term **"soluble"** or **"water soluble"** refers to an aqueous solubility that is higher than 1110,000 (mg/ml). The solubility of a substance, or solute, is the maximum mass of that substance that can be dissolved completely in a specified mass of the solvent, such as water. **"Practically insoluble"** or **"insoluble,"** on the other hand, refers to an aqueous solubility that is 1/10,000 (mg/ml) or less. Water soluble or soluble substances include, for example, polyethylene glycol.

The term **"substantially free"** means containing less than 1% by weight or as close to 0% of the composition as practicable. A composition is **"substantially free"** of a component, if the component was not added to the composition, but was otherwise present as an impurity or in trace amounts.

The term "purgative effective amount" or "purgative effective dosage" is used throughout the specification to describe the amount or concentration of PEG which is effective for producing a purgative effect, e.g., the elimination or evacuation from the intestines of its contents. In the case of PEG 8000, these have unexpectedly been found to be advantageously employed. The term "purgative active" is used to describe PEG according to the present invention which exhibit biological or pharmacological activity in the form of purgative activity.

### B. Description

Polyethylene glycol (PEG) 3350 is generally considered to act as an osmotic agent. It has been determined, surprisingly, that PEG of a higher molecular weight, such as PEG 8000, has beneficial purgative properties despite its osmolarity being lower than the osmolarity of known purgatives, such as PEG 3350. PEG 3350 (17 grams) has an osmolarity of 0.0465 Osm/L, whereas PEG 8000 (6.704 g) has an osmolarity of 0.0035 Osm/L, even after accounting for the difference in PEG used in this experiment. Thus, quite surprisingly, it has been realized that PEG polymers of a higher molecular weight, such as PEG 8000, would be beneficial in purging the colon.

It is expected that PEG 8000 is a superior purgative as it appears to act as a lubricant in the intestinal tract, making it easier to evacuate the contents of the colon. This lubricant effect is also very beneficial in performing a colonoscopy or other diagnostic or surgical procedures. It has been found that the endoscope is inserted more easily into the colon when PEG 8000 has been used, compared to prior compositions including PEG 3350, and that the walls of the intestine do not stick to each other as is often the case. This yields significant advantages for both the physician and the patient, as water or air does not need to be blown into the colon to allow the endoscope to pass deeper into the intestine. Any improvements in such a diagnostic or surgical procedure increase patient comfort, the likelihood that patients will undergo this procedure for cancer screening, and reduce the amount of time patients are under anesthesia. PEG 8000 may also function by sequestering water in the bowels in a way that does not register on an osmolarity test.

A further advantage is that the composition could be formulated in a variety of dosage forms. The composition, for instance, may be dispersed or dissolved in an aqueous substance before oral administration. Alternatively, the composition may be formulated in a solid dosage form, such as a tablet. Such a dual-dosage form composition would be desirable.

### 1. Polyethylene glycol

Any food- or pharmaceutical-grade PEG polymer may be employed in the compositions of the present invention. PEG is represented by the structural formula: HOCH₂(CH₂OCH2)mCH2OH, wherein m represents the average number of oxyethylene groups.

In one embodiment, the PEG polymers are solid at room temperature (e.g., 25°C) and/or soluble in (or miscible with) water at room temperature. In one embodiment, the average molecular weight of the PEG polymer is at least 1000, at least 4000, at least 4600, at least 6000, or at least 8000. In one embodiment, the average molecular weight of the PEG polymer is from 4000 to 35,000. In another embodiment, the PEG polymer has an average molecular weight of from 6000 to 20,000. In an additional embodiment the PEG polymer has an average molecular weight of 8000. In another embodiment, the PEG polymer has an average molecular weight of 3350.

In one embodiment, the composition is substantially free of electrolytes. In an alternative embodiment, the PEG composition may contain electrolytes, wherein the electrolytes do not adversely affect the osmotic action of the PEG polymer. Electrolytes that may be part of the composition include, but are not limited to, calcium, phosphate, potassium, magnesium, bicarbonate, chloride, sulfate, sodium, other cations, anions, or salts thereof, which may normally be lost in diarrhea fluid. These electrolytes also have an osmotic effect and may contribute to the purgative nature of the composition depending on the amounts included. If electrolytes are present in the composition, they may be included for a total of, for example, 5, 9.2, 10, 15, 18.4, 20, 25, 30, 35, 38.26, 38.31, 40,45, 50, 55, 60, 65, or 70 g. Examples of possible electrolytes that may be added to this PEG composition include, but are not limited to, four potential combinations:
Combination A:
   5.84 g sodium chloride
   2.98 g potassium chloride
   6.72 g sodium bicarbonate
   22.72 g sodium sulfate
Combination B:
   2.86 g sodium bicarbonate
   5.6 g sodium chloride
   0.74 g potassium chloride
Combination C:
   5.72 g sodium bicarbonate
   11.2 g sodium chloride
   1.48 g potassium chloride
Combination D:
   22.74 g sodium sulfate
   6.74 g sodium bicarbonate
   5.86 g sodium chloride
   2.97 g potassium chloride
In another embodiment, the composition is substantially free of any other types of purgatives and does not require administration with another type of purgative to achieve the desired laxative results (such as with a stimulant purgative or a bulk/fiber purgative) or complete purgation results (such with as a stimulant purgative).

### 2. Additional Optional Ingredients

Additional optional components may be included in the formulations of this invention to, for example, enhance the characteristics of the dosage form, maintain the integrity of particles of the active ingredient during the formulation process, and/or enhance the safety of the formulation. Any additional components may be compatible with the other ingredients in the formulations, in particular the active ingredients, and may not adversely affect the osmolarity of the formulations. Additional optional ingredients that may be used in the formulations include, for example, coatings, diluents, binders, glidants, lubricants, colorants, disintegrants, flavors, sweeteners, polymers or waxes.

Lubricants, for example, may be included in the formulations. Such lubricants include, but are not limited to, magnesium stearate, potassium stearate, talc, stearic acid, sodium lauryl sulphate, and paraffin. In one embodiment, the colonic purgative formulation further comprises magnesium stearate. Lubricants serve to facilitate the manufacturing of a solid dosage form.

Additional suitable ingredients also include, but are not limited to, carriers, such as sodium citrate and dicalcium phosphate; fillers or extenders, such as stearates, silicas, gypsum, starches, lactose, sucrose, glucose, mannitol, talc, and silicic acid; binders, such as hydroxypropyl methylcellulose, hydroxymethyl-cellulose, alginates, gelatin, polyvinyl pyrrolidone, sucrose, and acacia; humectants, such as glycerol; disintegrating agents, such as agar, calcium carbonate, potato and tapioca starch, alginic acid, certain silicates, colloidal silicon dioxide, sodium starch glycolate, crospovidone, and sodium carbonate; solution retarding agents, such as paraffin; wetting agents, such as cetyl alcohol and glycerol monostearate; absorbents, such as kaolin and bentonite clay; stabilizers, such as fumaric acid; coloring agents; buffering agents; dispersing agents; preservatives; organic acids; and organic bases.

Acidic or basic compounds may also be optionally added to the composition to adjust the pH of the composition or to alter the disintegration characteristics. Acidic or basic compounds that may be included in the formulations include, but are not limited to, sodium carbonate, sodium bicarbonate, sodium phosphate, calcium carbonate, magnesium hydroxide, potassium hydroxide, magnesium carbonate, and aluminum hydroxide.

Acidic or basic compounds may also be optionally added to the composition to form an effervescent component, in which the basic ingredient liberates carbon dioxide when it and the acidic ingredient are contacted with added water. In one embodiment, the acidic or basic compounds are citric acid or sodium hydrogen citrate and sodium bicarbonate. Other physiologically acceptable acidic or basic compounds include, but are not limited to, tartaric, adipic, fumaric or malic acids, and sodium, potassium or calcium (bi)carbonates or sodium glycine carbonate, or other acid/alkaline or alkaline earth metal carbonate mixtures.

The aforementioned ingredients are given as examples only and are not meant to include all possible choices. Additionally, many may have more than one role or function, or be classified in more than one group. Such classifications are descriptive only, and not intended to limit any use of a particular component.

To optimize the colonic purgative formulations, components and amounts of the colonic purgative formulations may be adjusted according to the knowledge of the person of ordinary skill in the art. Sample ingredient ranges for a colonic purgative formulation example are provided in Table 1. Not all of the components are necessary, but are provided for illustration only. For example, it may not be necessary to have electrolytes and it may also not be necessary to have a lubricant.

**Table 1: Example Ingredient Ranges for a Solid Dosage Colonic Purgative Composition**

| **Ingredient** | **Function** | **Qty % (w/w)** |
|---|---|---|
| PEG-8000 | Active | 50-90 |
| Optional Electrolyte | Active | 0-40 |
| Optional Magnesium Stearate | Tableting Lubricant | 0-1.50 |
| Optional Tablet Binders and Fillers | Tableting Agent | 0-10 |

### 3. Administration and Dosing

The present invention also encompasses methods of using the colonic purgative formulations. The colonic purgative formulations produce a broad range of activities, depending on the dosage administered. The present invention encompasses methods of purging the colon comprising administering to at least one patient a colonic purgative formulation and allowing said formulation to purge the colon. The formulations may also be used at lower doses in order to regulate, soften or loosen the stool.

Thus, the present invention also encompasses methods of maintaining the elimination or increasing the elimination of feces in the bowel, comprising administering to at least one patient a colonic purgative formulation and promoting the elimination of feces in the bowel. The colonic purgative formulations may also be used to treat a patient with constipation. The constipation may be caused by a variety of factors including, but not limited to at least one of travel; change in daily routine; lack of exercise; immobility caused by injury, illness, or aging; dehydration; irritable bowel syndrome; pregnancy; diabetes; hypothyroidism; hypercalcemia; cancer of the colon or rectum; uterine prolapse; vaginal vault prolapse; rectal prolapse; scarring from surgery; injury of the colon or rectum; Parkinson`s disease; multiple sclerosis; stroke; hemorrhoid or anal fissures; delaying bowel movements; anxiety; depression; eating disorders; and obsessive-compulsive disorder. The constipation may also be idiopathic, i.e. of unknown causation.

In another embodiment the composition is used to treat a patient suffering from, or susceptible to, constipation due to administration of a medication that causes constipation. A medication that may cause constipation includes, but is not limited to antacids that contain aluminum; antidepressants; blood pressure medications; calcium channel blockers; calcium supplements; chemotherapy medications; cold medicines; antihistamines; diuretics; iron supplements; medications for Parkinson's disease; lipid-lowering agents; pain medications; opiates; codeine; and tranquilizers.

One of skill in the art will recognize that the appropriate dosage of the colonic purgative compositions may vary depending on the individual being treated and the purpose. For example, the age, body weight, and medical history of the individual patient may affect the therapeutic efficacy of the therapy. Further, a lower dosage of the composition may be needed to produce a mild catharsis, while complete purgation may require a higher dose. A competent physician can consider these factors and adjust the dosing regimen to ensure the dose is achieving the desired therapeutic outcome without undue experimentation. It is also noted that the clinician and/or treating physician will know how and when to interrupt, adjust, and/or terminate therapy in conjunction with individual patient response. Dosages also may depend on the molecular weight of the particular PEG polymer chosen for the formulation.

In one embodiment, the total dosage is administered in at least one application period. In an additional embodiment, the total dosage is administered in two or more separate application periods, or separate doses.

The dose of the colonic purgative formulations may vary. For example, a lower dose of a colonic purgative formulation may be needed to produce a mild catharsis, while complete purgation may require a higher dose. A total daily dosage used for mild catharsis, for example, can range from 10 g to 100 g of a PEG polymer. For example, in general, a total daily dosage of a purgative, such as PEG 8000, in formulations of the present invention ranges from, for example, 10 to 100 g, 15 to 95 g, 17 to 90 g, 20 to 90 g, 25 to 85 g, 30 to 80 g, 40 to 70 g, 50 to 60 g, 25 to 75 g, or 35 to 65 g fora laxative effect. A total daily dosage may be formulated to contain, for example, 10, 15, 17, 20, 25, 30, 34, 35, 40, 50, 51, 60, 68, 70, 80, 85, 90, or 100 g of a purgative, such as PEG 8000. Additional doses *of the* colonic purgative formulation *may* be *necessary to* produce the desired therapeutic effect. In one embodiment, the total daily dosage is administered every 24 hours until the desired therapeutic effects are reached.

A higher dose of a colonic purgative formulation may be needed to produce a complete purgation of the colon. A total dosage used for complete purgation, for example, can range from 100 g to 1000 g of a purgative, optionally provided over a period of time of up to 24 hours. For example, in general, a total daily dosage of a PEG polymer, such as PEG 8000, in formulations of the present invention may range from 100 to 1000 g, 200 to 900 g, 300 to 800 g, or 400 to 700 g, 500 to 600 g for a complete purgative effect. A dose may be formulated to contain, for example, 100, 200, 210, 236, 240, 300, 400, 420, 500, 600, 700, 800, 900, 1000 g of a PEG polymer, such as PEG 8000. In an additional example, a total daily dosage of a PEG polymer, such as PEG 3350, in formulations of the present invention may range from, for example, 1 to 1000 g, 236 to 775 g, 240 to 750 g, 250 to 725 g, 350 to 600 g, or 400 to 450 g for a complete purgative effect. A dose may be formulated to contain, for example, 210, 236, 240, 400, 405, 410, 415, 420, 425, 430, 435 or 440 g of a PEG polymer, such as PEG 3350.

Optionally, in both the laxative and complete purgative embodiments, the total daily dosage may be separated into divided doses. In one embodiment, the total daily dosage is divided into two doses, separated by a period of up to 24 hours, for example. For instance, a total daily dosage of 500 g of a PEG polymer, such as PEG 8000, may be divided into two doses of 250 g each, as an example. One dose of 250 g may be administered in the evening before a colon procedure, while the second dose of 250 g may be administered in the morning, 3 to 6 hours before the colon procedure. In another embodiment, the total daily dose is divided into three, four, or more doses.

In one embodiment, the colonic purgative formulation is in an easily administered, solid dosage form. Solid dosage forms include, for example, a tablet, wafer, capsule, suppository, caplet, or sachet. The dosage form may be coated or encapsulated. In one embodiment, the colonic purgative formulation is incorporated into a food item. In another embodiment, the colonic purgative formulation is in the form of a tablet. The number of tablets administered in a dose may vary depending on the desired effect and on the amount of active ingredient in each solid dosage form. Clear liquids may be taken with each dose.

In another embodiment, the colonic purgative formulation is dispersed or dissolved in water or other aqueous medium. Suitable liquids include water, tea, or juice. In one embodiment, the colonic purgative composition is dissolved in water from, for example, 240 to 1000 ml for a laxative usage, including from, for example, 240 to 1000 ml, 480 to 800 ml, or 600 to 720 ml. The composition may be dissolved in water from 0.5 to 5 L for a complete purgative usage, including from, for example, 0.5 to 5 L, 0.75 to 4 L, 1 to 3 L, or 1.5 to 2 L. Other aqueous liquids may be used in a laxative preparation with no restrictions. In a complete purgation preparation, if the patient is undergoing a surgical or diagnostic procedure that could produce sparks, all fermentable liquids should be avoided, but the patient may use liquids containing an artificial sweetener such as diet drinks (Crystal Light^{™}, etc.) or water. The dose frequency for such a drink may be, for example, 240 ml every 10 minutes, 240 ml every 30 minutes, 480 ml every 30 minutes, or 1 L every hour, until the liquid is completely consumed.

In another embodiment the colonic purgative composition is dissolved in a smaller quantity of aqueous solution, such as from, for example, 240 to 1000 ml, 250 to 750 ml, or 480 to 500 ml, and the remaining liquid taken separately, but in conjunction with the composition.

In one embodiment, the colonic purgative composition is furnished in a solid dosage form for dispersal in a suitable liquid. In another embodiment, the colonic purgative composition is supplied in a pre-mixed liquid form. In an additional embodiment, the colonic purgative formulation is furnished in solid form for oral ingestion.

A colonic purgative composition may be part of a kit. In one embodiment, the kit further comprises materials to assist in the administration of the composition, for instance a cup or drinking container. In one embodiment, the kit comprises two, two-liter containers, wherein each two-liter container contains a colonic purgative formulation of polyethylene glycol having an average molecular weight of at least 1000, or at least 4000. In another embodiment, the kit comprises two, two-liter containers, wherein each two-liter container contains a colonic purgative formulation of PEG 3350. The PEG 3350 may be present in a dose of from 200 to 400 g in each container. In one embodiment, the PEG 3350 is present in a dose of 210 g in each two-liter container. In yet another embodiment, the kit comprises two, two-liter containers, wherein each two-liter container contains a colonic purgative formulation of PEG 8000. The two, two-liter containers may optionally be packaged together in a box, a bag, or with shrink wrap and the like.

Additionally, in another embodiment, the jugs are of a size and shape that is easy to fit into a crowded refrigerator. For example, the jugs may be prepared so they could fit in the refrigerator door. In one embodiment, the jugs may be stacked one on top of the other. Optional grooves may facilitate the stacking. The jugs may be stacked in an upright position, or by turning them on their side. They may be packaged one on top of the other, or side by side.

The kit may contain optional ingredients in addition to the purgative, such as at least one electrolyte in each two-liter container. In another embodiment, the kit further comprises a flavoring agent in each two-liter container. The flavoring agent may also be supplied in the kit as a separate package that is added to the container prior to reconstitution of the purgative with an aqueous substance.

A colonic purgative composition may be administered by various routes. In one embodiment, the purgative composition is administered orally. In an additional embodiment, the purgative composition is administered through a tube, for instance a feeding tube or nasogastric tube. In another embodiment, the purgative composition is administered rectally.

### C. Examples

The following examples are offered for illustrative purposes only.

### Example 1: Osmolarity of colonic purgative compositions

The osmolarity (Osm/kg H₂O concentration) of four aqueous solutions constituted from solids was determined using an Osmette S Automatic^{®} (Precision Systems Inc., Sudbury, MA), freezing point osmometer. Each solution was prepared by accurately weighing the component(s) listed in Table 2, which were provided in powder or tablet form. The component(s) of each sample were transferred to a 500 ml beaker and subsequently 240 ml of water was added to the beaker. Each sample was then gently agitated until the soluble components were dissolved. As expected, the Visicol^{®} (lnKine Pharmaceutical Company, Blue Bell, PA) sample contained undissolved solid.

The freezing point osmometer was calibrated by a two point procedure, using 100 and 500 mOsm/L standards. The osmolarity of each sample was measured in duplicate and the results averaged. The average osmolarity of each sample is presented in Table 2. Theoretical calculations of osmolarity, as shown in Table 2, were based on colligative behavior, assuming 80% dissociation for all electrolytes, and completed dissolution of polyethylene glycol, a non-ionic molecule.

**Table 2: Osmolarity of Colonic Purgative_Compositions**

| **Sample** | **Osmolarity (Osm/L)*** | | **Components** |
|---|---|---|---|
| | **Theoretical** | **Actual**** | |
| **Phosphate Salts** | **0.3610** | 0.3305 | 4.408 g Sodium phosphate monobasic monohydrate; 1.592 g Sodium phosphate dibasic anhydrous |
| **Visicol^{®}** | NIA | 0.3285 | 4.408 g Sodium phosphate monobasic monohydrate; 1.592 g Sodium. phosphate dibasic anhydrous; 0.918 g Microcrystalline cellulose; 0.0353 g Colloidal silicone dioxide; 0.106 g Magnesium stearate |
| **PEG 8000** | **0.0035** | **0.0035** | 6.704 g PEG 8000 |
| **PEG 3350** | 0.0211 | **0.0465** | 17.0 g PEG 3350 |

| | | | |
|---|---|---|---|
| * Resolution of 0.001 Osm. ** Average of two measurements. | | | |

Actual osmolarity values obtained for all of the samples approximated the expected osmolarity values, with the exception of PEG 3350. The PEG 3350 solution had an actual osmolarity about two times greater than the expected value. This could be due, in part, to the average molecular weight of the PEG being less than the 3350 value used in the theoretical calculation. This is unlikely, however, because the sample was manufactured according to Good Manufacturing Practice (GMP). The increased actual osmolarity of PEG 3350 may also reflect strong hydration of the polymer, effectively increasing its effect on freezing point depression and observed osmolarity.

The actual osmolarity of PEG 8000 was relatively low compared to the other colonic purgative solutions and was approximately 13 times smaller than the actual osmolarity of PEG 3350, despite the fact that only 2.5 times more PEG 3350 was used. One would therefore expect PEG 8000 to have much less, if any, purgative effect compared to a similar amount of PEG 3350.

### Example 2: Colon cleansing efficacy of polyethylene glycol 8000

The primary objective of this study was to examine the purgative effect of a composition of one embodiment, PEG 8000, versus inactive vehicle (Crystal Light^{®}; lemonade diet drink reconstituted in water; Kraft Foods North America, Inc., Rye Brook, NY) in normal healthy subjects. In addition, the safety and tolerability of the PEG 8000 was evaluated.

### Treatments

This study was a double-blinded, randomized, crossover study with 16 healthy male subjects. Subjects were randomly assigned to one of two dosing sequences: Sequence A (n=9) or Sequence B (n=6). For each dosing sequence there were three scheduled visits: a screening visit (Visit 0) and two treatment visits (Visits 1 and 2). After the initial screening visit, both dosing sequences began with a 33 hour period in which subjects were maintained on a standardized *2000 calorie, 22 g fiber per day diet. See* Table *3*. *At the beginning* of day 4, the standardized diet ended and subjects were given a low fiber breakfast followed by clear liquids for the rest of the day. On day 5, patients received nothing by mouth (NPO) after the administration of the test materials.

A baseline period measurement period began at 6 p.m. on day 2 and lasted 48 hours. During the baseline period, all bowel movements were collected, recorded, and assessed for consistency and weight. At 6 p.m. on day 4, the test article administration began. Sequence A was administered PEG 8000 in the inactive vehicle during the treatment period of Visit 1, and only the inactive vehicle during the treatment period of Visit 2. Sequence B was administered the inactive vehicle only during the treatment period of Visit 1, then PEG 8000 in the inactive vehicle during the treatment period of Visit 2. In both dosing sequences, Visit I was followed by a two-week washout period before subjects crossed over to begin visit 2. During the observation period of test article administration, all bowel movements were collected, recorded, and assessed for consistency and weight. The observation period of test article administration began when the test article was administered and continued until 11 a.m. on day 5.

**Table 3: Study Design for both Visit 1 and 2 and for both Sequence A and B**

| | **Day 1** | **Day 2** | **Day 3** | **Day 4** | **Day 5** |
|---|---|---|---|---|---|
| **Diet** | 9:00 am. | Standard diet continues | Standard diet continues | 9:00 a.m. | NPO after administration of test article |
| | Standard diet begins | | | Standard diet ends; Low fiber breakfast and clear liquids begin | |
| **Baseline** | | 6:00 p.m. | Baseline period continues | 6:00 p.m. | |
| | | Baseline period begins | | Baseline period ends | |
| **Test Article Administration and Observation** | | | | 6:00 p.m. | 6:00 a.m. |
| | | | | Test article administered and Observation period begins | Test article administered 11:00 a.m. Observation period ends |

The PEG 8000 composition administered to the subjects was dissolved in diluted inactive vehicle (Crystal Light^{®}; lemonade diet drink reconstituted in water; Kraft Foods North America, Inc., Rye Brook, NY). A separate, double-blind test of the inactive vehicle with or without PEG 8000 demonstrated that the two solutions were indistinguishable by appearance, taste, odor, or mouth feel. For the current study, one tub of inactive vehicle was mixed in 4 quarts of water so that the solution had one-half of the normal concentration suggested on the packaging. The PEG 8000 solution was prepared such that 8 oz. of inactive vehicle contained approximately 670.4 mg PEG 8000, NF. Subjects drank 8 oz. of this preparation every 15 minutes over 1 hour beginning at 6:00 p.m. on day 4 (total of 5 doses, containing 3.352 g PEG 8000). Subjects drank another 5 doses in the same manner beginning at 6:00 a.m. on day 5. Thus, the total dose of PEG 8000 administered to each subject was 6.704 g, delivered in 80 oz. of inactive vehicle. During the inactive vehicle only phase, each subject was given the inactive vehicle without dissolved PEG 8000 on the same schedule.

### Efficacy: Primary Endpoint

Fifteen patients passed screening and completed both visits of the study. One patient was removed from the study after becoming combative and non-compliant with study requirements during Visit 2.

The primary endpoint for efficacy was the difference in total wet stool weights collected between the Baseline and Observation periods, adjusted for the differing durations of these two periods. Specifically, as the baseline period was 48 hours long and the observation period was only 17 hours long, this data has been time adjusted to yield a total fecal weight per day (24 hour period). Table 4 displays the mean total fecal weight measurements by test article in the 15 patients. Comparison of PEG-8000 versus inactive vehicle treatments revealed that there was a small baseline-to-PEG 8000 increase in daily fecal weight (mean change= +3.4 g). At the same time, there was a decrease in daily fecal weight from baseline-to-inactive vehicle (mean change= -60.8 g). The decrease in the baseline-to-inactive vehicle may be in part attributable to 10 subjects who did not have a bowel movement in the Observation period after receiving the inactive vehicle. Data were also collected based on the sequence patients took the test articles, PEG then vehicle or vehicle then PEG. Both data sets were analyzed for statistical significance.

**Table 4: Total Fecal Weight (g) Per Day by Test Article**

| **Test Article** | **Mean (SD) Fecal Weight** | | |
|---|---|---|---|
| | **Baseline** | **Observation** | **Change (Baseline- Observation)** |
| **PEG 8000 (n=15)** | 88.2 (50.0) | 91.7 (87.3) | +3.4 (114.0) |
| **Inactive Vehicle (n=15)** | 106.1 (71.5) | 45.3 (71.2) | -60.8 (123.2) |

| | | | |
|---|---|---|---|
| **SD= standard deviation** | | | |

Statistical analysis of the primary endpoint was accomplished by using a linear model implemented in SAS/ STAT PROC MIXED, with change from Baseline period to Observation period in adjusted daily total fecal weight as the outcome, taking into account both the summary test article data and the sequence of the test article. The test article and sequence (PEG 8000 or inactive vehicle first) were model terms. With 13 degrees of freedom, the statistical analysis determined that the change in adjusted daily total fecal weight was not significant (p=0.12 for the PEG 8000 effect; p=0.49 for the sequence effect). A secondary model was constructed that included terms for age, body weight, and body mass index as covariates. However, even after adjustment for these demographic covariates, there was still no statistical significance.

### Efficacy: Secondary Endpoints

Three secondary endpoints were also considered: per-bowel movement fecal weight, number of bowel movements, and Bristol Stool Consistency Scale. Statistical analysis of the secondary endpoints was accomplished as described above for the primary endpoint. However, the outcome in the linear model was either per-bowel movement fecal weight, number of bowel movements, or Bristol Stool Consistency Scale.

### Per-Bowel Movement Fecal Weight

In this analysis, a measurement for each data collection period, an average fecal weight per bowel movement (total fecal weight divided by the number of bowel movements) was determined for each subject. See Table 5. When a subject had no bowel movements in a given time period, no data were contributed for that collection period, leading to substantial variation in sample sizes, making this data more difficult to interpret. There were no significant differences shown in per bowel movement weight. Further, it is difficult to evaluate this data because if a patient happens, by chance, to have an especially large bowel movement during the observation period, it is less likely that they will have another large bowel movement during the study due to the reduced contents of the bowel and an individual's own bowel frequency. The sample size was not large enough to account for these possible irregularities in the data.

**Table 5: Per-Bowel Movement Fecal Weight by Test Article**

| **Test Article** | **Per Bowel Movement Fecal Weights (SD)** | | |
|---|---|---|---|
| | **Baseline for Evaluable Patients** | **Observation** | **Change (Baseline-Observation)** |
| **PEG 8000 (n=11)** | 98.5 (43.9) | 81.9 (58.8) | -16.7 (72.0) |
| **Inactive Vehicle (n=5)** | 67.7 (40.5) | 96.1 (34.6) | 28.4 (32.9) |

| | | | |
|---|---|---|---|
| **SD= standard deviation** | | | |

### Number of Bowel Movements

In this study, the number of bowel movements were counted for each patient. See Table 6. As the baseline period was 48 hours long and the observation period was only 17 hours long, this data has been time adjusted to yield a number of bowel movements per day (24 hour period). PEG 8000 was associated with a higher frequency of bowel movements than was the inactive vehicle, showing that even at these low doses PEG 8000 increases the number of bowel movements.

**Table 6: Number of Bowel Movements Per Day**

| **Test Article** | **Number of Bowel Movements (SD)** | | |
|---|---|---|---|
| | **Baseline for Evaluable Patients** | **Observation** | **Change (Baseline-Observation)** |
| **PEG 8000 (n=15)** | 0.9 (0.4) | 1.2 (0.9) | 0.3 (0.9) |
| **Inactive Vehicle (n=15)** | 1.0 (0.4) | 0.5 (0.7) | -0.5 (0.9) |

| | | | |
|---|---|---|---|
| SD= standard deviation | | | |

Statistical analysis for this secondary endpoint was performed. The statistical analysis determined that the change in number of bowel movements was significant (p=0.019, without covariate adjustment) in a test for superiority of PEG-8000 over the inactive vehicle. An additional analysis was performed that included terms for age, body weight, and body mass index as covariates. After adjustment for these demographic covariates, the p-value was also significant (p=0.022).

### Bristol Stool Consistency Scale

The Bristol Stool Consistency Scale is used to evaluate the consistency of a patients stool on an ordinal scale with possible values from 1-7 with higher values indicating looser stool with the upper limit representing watery stools. See Table 7. In the absence of a bowel movement, this value remains undefined, providing variation in the sample sizes for this measure. Thus, due to the variation in sample size, it is more difficult to interpret these results. Therefore, the fact that the inactive vehicle was more associated with watery stool cannot be considered a reliable conclusion. Additionally, this was not shown to be a statistically significant difference.

**Table 7: Bristol Stool Consistency Scale**

| **Test Article** | **Bristol Stool Consistency Scale (SD)** | | |
|---|---|---|---|
| | **Baseline for Evaluable Patients** | **Observation** | **Change (Baseline-Observation)** |
| **PEG 8000** (n=11) | 3.7 (1.0) | 3.1 (0.8) | -0.5 (1.3) |
| **Inactive Vehicle (n=5)** | 3.0 (1.0) | 3.4 (0.9) | 0.4 (1.5) |

| | | | |
|---|---|---|---|
| SD= standard deviation | | | |

### Efficacy: Conclusions

Administration of PEG 8000 produced a significant increase in bowel movements, .and a nonsignificant increase in fecal weight, even though the osmolarity of PEG 8000 was lower than that of other colonic purgative compositions, such as PEG 3350. See Example 1. It did not show an effect in per-bowel movement fecal weight or the Bristol Stool Consistency scale. It is therefore expected that, at larger doses, PEG 8000 will be an effective colonic purgative composition, even though it is not an effective colonic purgative at the dose provided in this study (as measured either by the primary efficacy parameter or all of the efficacy parameters). Suggestions of its activity may be found in this data, even though it is not effective at the dose provided. It is expected that PEG 8000, either alone or with electrolytes, may be used as a colonic purgative (for either laxative or complete purgative uses) at higher dose than those presented here (i.e., higher than 670.4 mg). It is expected that at higher doses, all four parameters will show increases in purgative activity.

### Safety

No adverse events were reported during the period between the first dose (6:00 p.m. day 4) and 72 hours after the last dose (7:00 a.m. day 5) in each visit. Three minor adverse events were reported outside of the treatment period. Two subjects reported mild headaches and one subject displayed moderate flatulence, which was unrelated to the test article. There were no serious events, no severe events, and no deaths. Finally, there were no episodes of orthostatic hypertension, and no notable or clinically significant findings in regard to vital signs, physical examination, or use of concomitant medications.

### Example 3: A colonic purgative composition containing

### polyethylene glycol to purge the colon

A patient undergoing a surgical or diagnostic procedure involving the colon is administered 100-1000 g of a colonic purgative composition comprising a polyethylene glycol 8000. The evening before the surgical or diagnostic procedure, 100-1000 g are taken dissolved or dispersed in at least 240 ml to 4 L of an aqueous solution total, such as water, in either one dose or divided doses with larger volumes of solution requiring divided doses. Optionally, the day of the colonoscopy procedure, (starting 3 to 6 hours before the procedure) 100-500 g are taken dissolved or dispersed in at least 240 ml to 2 L of an aqueous solution, such as water. It is expected that the results of such treatment will provide an adequately cleansed bowel, is tolerable to the patient both in its palatability and side effect profile, and will allow for more gentle insertion of an endoscope or other medical instrument.

### Example 4: A colonic purgative composition containing

### polyethylene glycol to treat constipation

A patient with constipation is treated with a colonic purgative composition comprising polyethylene glycol 8000. The composition is administered in a total daily dose of 10 to 100 g and is administered in a tablet or capsule dosage form. The dose may be repeated daily. It is expected that the results of such treatment will facilitate the passage of feces and promote elimination, by loosening or softening of the stool and/or the promotion of peristalsis due to increased amounts of water in the colon. It is also expected that the results of such treatment will be tolerable to the patient both in its palatability and side effect profile. It is beneficial to be able to use the same dual function composition for complete purgation and laxation.

### Example 5: A kit containing a colonic purgative composition

A patient undergoing a surgical or diagnostic procedure involving the colon is supplied with a kit. The kit contains two, two-liter jugs. Each two-liter jug contains a colonic purgative composition comprising 240 g polyethylene glycol 3350,5.84 g sodium chloride, 2.98 g potassium chloride, 6.72 g sodium bicarbonate, and 22.72 g sodium sulfate. Optionally, each jug also contains a flavoring agent, flavoring agents may be added by the patient *(such as* Crystal Light*^{™})*, *or flavoring agents may* be supplied *in a separate* package by the manufacturer in either one flavor choice or multiple flavor choices. The day of the colonoscopy procedure (starting 4 to 5 hours before the procedure), each jug is filled to the two-liter fill mark with water and shaken to dissolve the solid composition. The patient drinks the aqueous purgative composition at a rate of 8 oz. (240 ml) every 10 minutes until the 4 liters of liquid is consumed. It is expected that the results of such treatment will provide an adequately cleansed bowel, is tolerable to the patient both in its palatability and side effect profile, and will allow for more gentle insertion of an endoscope or other medical instrument. Further, it is expected that the two, two-liter jugs will not be as psychologically daunting to the patient as a larger container and will therefore increase patient compliance. Additionally, it is expected that the two, two-liter jugs will be easier to handle, especially for elderly, infirm, or handicapped patients.

All references cited herein are incorporated herein by reference in their entirety and for all purposes to the same extent as if each individual publication or patent or patent application was specifically and individually indicated to be incorporated by reference in its entirety for all purposes. To the extent publications and s or applications incorporated by reference contradict the disclosure contained in the specification, the specification is intended. to supercede and/or take precedence over any such contradictory material.

All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit being indicated by the following claims.

## Claims

1. A colonic purgative formulation comprising a therapeutically effective amount of polyethylene glycol having an average molecular weight of at least 4000 and optional electrolytes for use in treating a gastrointestinal disorder by providing the formulation as a laxative.

2. The formulation for use according to claim 1, wherein the polyethylene glycol comprises an average molecular weight of from 4000 to 20000.

3. The formulation for use according to claim 1, wherein the polyethylene glycol comprises PEG 8000.

4. The formulation for use according to claim 1, comprising from 10 to 100 g of PEG 8000 and optional electrolytes.

5. The formulation for use according to claim 1, wherein the gastrointestinal disorder is constipation.

6. The formulation for use according to claim 5, wherein the constipation is due to administration of a medication which causes constipation as a side effect.

7. The formulation for use according to claim 6, wherein the medication is chosen from antacids that contain aluminum; antidepressants; blood pressure medications; calcium channel blockers; calcium supplements; chemotherapy medications; cold medicines; antihistamines; diuretics; iron supplements; medications for Parkinson's disease; lipid-lowering agents; pain medications; opiates; codeine; and tranquilizers.

8. The formulation for use according to claim 6, wherein the constipation is due to at least one of travel; change in daily routine; lack of exercise; immobility caused by injury, illness, or aging; dehydration; irritable bowel syndrome; pregnancy; diabetes; hypothyroidism; hypercalcemia; cancer of the colon or rectum; uterine prolapse; vaginal vault prolapse; rectal prolapse; scarring from surgery; injury of the colon or rectum; Parkinson's disease; multiple sclerosis; stroke; hemorrhoids or anal fissures; delaying bowel movements; anxiety; depression; eating disorders; and obsessive-compulsive disorder.

9. A colonic purgative formulation comprising a therapeutically effective amount of polyethylene glycol having an average molecular weight of at least 4000 and optional electrolytes for use in preparing the colon for a colonic procedure by providing the formulation as a complete purgative agent.

10. The formulation for use according to claim 9, comprising from 100 to 1000 g of PEG 8000 and optional electrolytes.

11. The formulation for use according to claim 9, wherein the polyethylene glycol comprises an average molecular weight of from 4000 to 20000.

12. The formulation for use according to claim 9, wherein the polyethylene glycol comprises PEG 8000.

13. The formulation for use according to claim 9, wherein the colonic procedure is a colonoscopy.

14. The formulation for use according to claims 1 or 9, wherein the colonic purgative formulation is in a solid dosage form.

15. The formulation for use according to claims 1 or 9, wherein the colonic purgative formulation is a tablet.

16. The formulation for use according to claims 1 or 9, comprising between 50 w/w% to 90 w/w% PEG-8000.
